(19) Europäisches Patentamt
European Patent Office
Office européen des brevets

(11) Veröffentlichungsnummer: **0 107 140**
**B1**

# (12) EUROPÄISCHE PATENTSCHRIFT

(45) Veröffentlichungstag der Patentschrift: **16.03.88**

(51) Int. Cl.[4]: **C 12 P 7/02**

(21) Anmeldenummer: **83110122.5**

(22) Anmeldetag: **11.10.83**

## (54) Verfahren zur Herstellung von p-Menth-8-en-1,2-diol.

(30) Priorität: **26.10.82 DE 3239545**

(43) Veröffentlichungstag der Anmeldung: **02.05.84 Patentblatt 84/18**

(45) Bekanntmachung des Hinweises auf die Patenterteilung: **16.03.88 Patentblatt 88/11**

(84) Benannte Vertragsstaaten: **CH DE FR GB LI NL**

(56) Entgegenhaltungen:
**EP-A-0 012 246**
**US-A-3 607 651**

**CHEMICAL ABSTRACTS, Band 66, Nr. 7, 13. Februar 1967, Seite 2515, Ref. 26728f, Columbus, Ohio, US; R.S. DHAVALIKAR et al.: "Microbiological transformation of terpenes. VIII. Fermentation of limonene by a soil pseudomonad" & INDIAN J. BIOCHEM. 3(3), 144-57 (1966)**
**CHEMICAL ABSTRACTS, Band 78, Nr. 19, 14. Mai 1973, Seite 206, Ref. 121159c, Columbus, Ohio, US; B.B. MUKHERJEE et al.: "Synthesis of glycols by microbial transformation of some monocyclic terpenes" & APPL. MICROBIOL. 1973, 25(3), 447-53**
**CHEMICAL ABSTRACTS, Band 85, Nr. 3, 19. Juli 1976, Seite 504, Ref. 19027r, Columbus, Ohio, US; E.R. BOWEN: "Potential by-products from microbial transformation of d-limonene" & PROC.**

(73) Patentinhaber: **Gesellschaft für Biotechnologische Forschung mbH (GBF), Mascheroder Weg 1, D-3300 Braunschweig- Stöckheim (DE)**

(72) Erfinder: **Abraham, Wolf- Rainer, Dr., Kleegasse 2, D-3300 Braunschweig (DE)**
Erfinder: **Stumpf, Burkhard, Dr., Sonnenweg 4, D-3308 Königslutter (DE)**
Erfinder: **Kieslich, Klaus, Prof.Dr., Fischhausenweg 4, D-3300 Braunschweig (DE)**

(74) Vertreter: **Boeters, Hans Dietrich, Dr., Boeters, Bauer & Partner Thomas- Wimmer- Ring 14, D-8000 München 22 (DE)**

(56) Entgegenhaltungen: (Fortsetzung)
**FLA. STATE HORTIC. SOC. 1976, 88, 304-8**

EP 0 107 140 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Mikrobiologische Umwandlungen von Limonen sind seit längerer Zeit bekannt[1]. Die beschriebenen Reaktionen waren jedoch zunächst Resultate von Untersuchungen des Abbaus von nicht eindeutig beschriebenen Limonen mit Pseudomona-den[2-8] und Enterobacteriaceae[9,10]. Das Substrat diente hierbei als Kohlenstoff- und Energiequelle dieser Mikroorganismen. Aspergillus niger NCIM 612 oxidiert Limonen zu (+)-cis-Carveol, alpha-Terpineol und Folgeprodukten[11]. Alle Metabolite konnten jedoch nur in geringen Substanzmengen als Intermediärprodukte des Abbaus isoliert werden.

Ein auf Kohlenwasserstoffen gezüchteter Stamm von Corynebacterium hydrocarboclastus oxidiert dagegen das Cosubstrat Limonen zu Carvon, welches in präparativer Ausbeute gewonnen wurde[12].

Cis- und trans-p-Menth-8-en-1,2-diol wurden bisher nur ohne Zuordnung zur 4-(S)- oder 4-(R)-Strukturreihe als Zwischenstufen des bakteriellen Limonenabbaus beschrieben[9].

Erfindungsgemäß wurde nun festgestellt, daß sich bei Biotransformationen von (R)(+)-Limonen das definierte (1S, 2S, 4R)-p-Menth-8-en-1,2-diol präparativ herstellen läßt. Für diese Umwandlung sind verschiedene Fungi der Gattungen Fusarium, wie Fusarium oxysporum DSM 62297; Gibberella, wie Gibberella cyanea DSM 62719 und Gibberella heterochroma DSM 62720; Chaetomium, wie Chaetomium globosum DSM 62109 und Chaetomium cochliodes DSM 1909; Diplodia, wie Diplodia oleae CBS 17453 und Diplodia gossypina ATCC 10936; Corynespora, wie Corynespora cassiicola DSM 62474 und DSM 62475 und Corynespora melonis CBS 12925; Glomerella, wie Glomerella miyabeana DSM 62731, Glomerella capsulata DSM 1166 und Glomerella cingulata ATCC 12097; Aspergillus, wie Aspergillus sclerotiorum DSM 63357 und Aspergillus caespitosus CBS 10345; Pestalotia, wie Pestalotia microspora ATCC 11816; Streptomyces, wie Streptomyces rimosus NRRL 2234; Botryodiplodia, wie Botryodiplodia theobromae DSM 62078; Botryosphaeria, wie Botryosphaeria berengeriana ATCC 12577 geeignet. Bevorzugt sind Diplodia gossypina ATCC 10936 und Corynespora cassiicola DSM 62474 und DSM 62475, wobei teilweise nur geringe Mengen an Nebenprodukten gebildet werden.

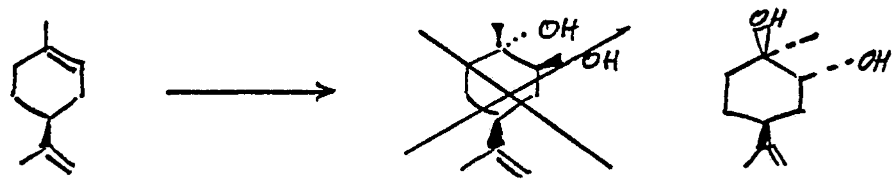

In gleicher Art wird (S)(-)-Limonen durch dieselben Fungi in präparativen Ausbeuten zum definierten (1R, 2R, 4S)-p-Menth-8-en-1,2-diol umgewandelt.

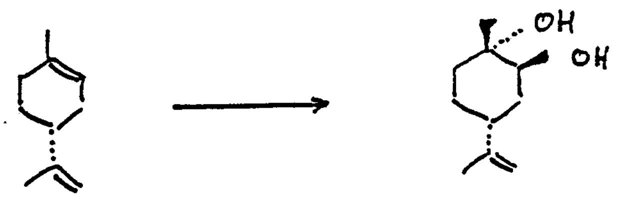

In gleicher Weise, jedoch bevorzugt mit Diplodia gossypina und Corynespora cassiicola wird auch racemisches Limonen, d.h. die Mischungen von R(+)-Limonen und S(-)-Limonen, zu entsprechenden Gemischen von (1S, 2S, 4R)- und (1R, 2R, 4S)-p-Menth-8-en-1,2-diol umgewandelt.

Die dabei anzuwendenden Fermentationsbedingungen entsprechen dem Stand der Technik[1].

Neben den speziell angegebenen Species und Stämmen ist es dem Fachmann routinemäßig möglich (insbesondere anhand der in Beispiel 1 beschriebenen Farbreaktion des p-Menth-8-en-1,2-diols) weitere brauchbare Species und weitere brauchbare Stämme zu ermitteln.

Die nach diesen Verfahren herstellbaren Substanzen sind Ausgangspunkte für weitere chemische Synthesen, was z. B. durch Dehydratisierung zu den entsprechenden cis-Mentha-2,8-dien-1-olen belegt wird, die Ausgangsstrukturen für Tetrahydrocannabinole darstellen[13].

**Beispiel 1**

Für die Vorkultur wird zunächst ein 100 ml Erlenmeyerkolben mit 20 ml sterilisiertem Kulturmedium, enthaltend 1 % D(+)-Glucose, 2 % Malzextrakt, 1 % Universalpepton (Merck) und 0,5 % Hefeextrakt, mit 3 Impfösen einer ausgewachsenen (7 Tage) Schrägagarkultur von Corynespora cassiicola DSM 62475 beimpft und 72 h bei 27°C unter Schütteln im Dunkeln inkubiert.

Anschließend wird diese Kultur unter aseptischen Bedingungen in einen 2000 ml Erlenmeyerkolben mit 400 ml Kulturmedium überführt und 48 h unter oben angegebenen Bedingungen kultiviert.

Da dieser Organismus in Submerskultur dazu neigt, die Kulturlösung nicht homogen zu durchwachsen, sondern vielmehr unterschiedlich geformte "Mycelklumpen" ausbildet, wird die Vorkultur mit Hilfe eines

sterilisierbaren, magnetischen Schneidegeräts[14] im Schüttelkolben zerkleinert und weiter 24 h inkubiert.

Aus dieser Vorkultur werden fünf 2000 ml Fermenterkolben, die ebenfalls 400 ml Kulturmedium enthalten, mit jeweils 20 ml beimpft und 64 h vorfermentiert. Danach wird jedem Kolben 85 mg R(+)-Limonen zugesetzt. Nach jeweils 8 h wird noch 2mal 85 mg Edukt hinzugefügt, und nach einer Gesamtkontaktzeit von 48 h wird die Fermentation nach vollständigem Umsatz beendet.

Der Verlauf der Umwandlung wird dünnschicht-chromatographisch verfolgt. Dazu wird 1 ml einer steril entnommenen Probe mit 0,2 ml Ethylacetat versetzt, 2 min geschüttelt und 2 min zentrifugiert. Vom Überstand werden 0,01 ml auf HPTLC-Fertigplatten, Kieselgel Si-60 (Merck) aufgetragen und 5 cm mit Dichlormethan/Aceton (80 + 20) entwickelt.

Anschließend wird die Platte mit folgendem Reagens besprüht:

| | |
|---|---|
| 0,6 ml | Anisaldehyd |
| 1,0 ml | Schwefelsäure |
| 50,0 ml | Essigsäure |

Zur Sichtbarmachung der Flecken wird 2 min bei 100° C inkubiert.

Für die präparative Aufarbeitung werden Überstand und Mycel mit Ethylacetat extrahiert und auf 5 ml eingeengt.

Die chromatographische Trennung erfolgt an einer Kieselgelsäule Si-60 (Merck) (2 cm x 35 cm), die gegen Dichlormethan äquilibriert ist und nach Probenauftrag mit einem Gradienten von 5 - 10 % Aceton in Dichlormethan entwickelt wird.

Es werden 1,27 g (1S, 2S, 4R)-p-Menth-8-en-1,2-diol (80 % d.Th) erhalten.

**Beispiel 2**

Dieses Beispiel unterscheidet sich von Beispiel 1 lediglich dadurch, daß anstelle von R(+)-Limonen nunmehr S(-)-Limonen eingesetzt wird.

Es werden 1,21 g (1R, 2R, 4S)-p-Menth-8-en-1,2-diol (76 % d.Th) erhalten.

**Beispiel 3**

Dieses Beispiel unterscheidet sich von Beispiel 1 nur dadurch, daß anstelle von R(+)-Limonen dl-Limonen (Dipenten) eingesetzt wird.

Es werden 1,23 Gemisch der enantiomeren Diole der Beispiele 1 und 2 (77 % d.Th) erhalten.

Literatur

1) K. Kieslich
Microbial Transformations of Non-Steroid Cyclic Compounds
Georg Thieme, Stuttgart 1976

2) R.S. Dhavalikar, P.N. Rangachari, P.K. Bhattacharyya
Ind. J. Biochem. 3, 158 (1966)

3) N.R. Ballal, P.K. Bhattarharyya, P.N. Rangachari
Biochem. Biophys. Res. Comm. 29, 275 (1967)

4) N.R. Ballal, P.K. Bhattacharyya, P.N. Rangachari
Biochem. Biophys. Res. Comm. 23, 473 (1966)

5) N.R. Ballal, P.K. Bhattacharyya, P.K. Rangachari
Ind. J. Biochem. 5, 1 (1968)

6) B.L. Hugund, P.K. Bhattacharyya, P.N. Rangachari
Arch. Microbiol. 71, 258 (1970)

7) B.L. Hugund, P.K. Bhattacharyya, P.N. Rangachari.
Ind. J. Biochem. 7, 80 (1970)

8) J.R. Devi, P.K. Bhattacharyya
Biochem. Biophys. 14, 359 (1977)

9) R.S. Dhavalikar
Ind. J. Biochem. 3, 144 (1966)

10) S.G. Dhere, R.S. Dhavalikar
Sci. Cult. 36, 292 (1970)

11) G.K. Swamy, K.L. Dhanchandani, P.K. Bhattacharyya
Abstr. Sympos. on Recent Advances in the Chemistry of Terpenoids
(Nation. Inst. of Sciences of India, New Delhi) (1965), 10.

12) Hasegawa Co.
Jap. Pat. 7 238998 (1972)

13) Hasegawa Co.
Aust. J. Chem. 33, 451 (1980)

14) B. Stumpf. L. Heuer
GBm-Anmeldung G 8216 356.1

**Patentansprüche**

1. Verfahren zur Herstellung eines Gemischs aus (1S, 2S, 4R)-p-Menth-8-en-1,2-diol und (1R, 2R, 4S)-p-Menth-8-en-1,2-diol, dadurch gekennzeichnet, daß man racemisches Limonen mit p-Menth-8-en-1,2-diol erzeugenden Fungi der Gattungen Fusarium, Gibberella, Chaetomium, Diplodia, Corynespora, Glomerella, Aspergillus, Pestalotia, Streptomyces, Botryodiplodia, Botryosphaeria oder deren Enzymen fermentiert und das genannte Gemisch gewinnt.

2. Verfahren zur Herstellung von (1S, 2S, 4R)-p-Menth-8-en-1,2-diol, dadurch gekennzeichnet, daß man (R)(+)-Limonen mit p-Menth-8-en-1,2-diol erzeugenden Fungi der Gattungen Fusarium, Gibberella, Chaetomium, Diplodia, Corynespora, Glomerella, Aspergillus, Pestalotia, Streptomyces, Botryodiplodia, Botryosphaeria oder deren Enzymen fermentiert und das genannte Diol gewinnt.

3. Verfahren zur Herstellung von (1R, 2R, 4S)-p-Menth-8-en-1,2-diol, dadurch gekennzeichnet, daß man (S)(-)-Limonen mit p-Menth-8-en-1,2-diol erzeugenden Fungi der Gattungen Fusarium, Gibberella, Chaetomium, Diplodia, Corynespora, Glomerella, Aspergillus, Pestalotia, Streptomyces, Botryodiplodia, Botryosphaeria oder deren Enzymen fermentiert und das genannte Diol gewinnt.

4. Verfahren nach Anspruch 1, 2 oder 3, dadurch gekennzeichnet, daß man mit Diplodia gossypina ATCC-10936 oder Corynespora cassiicola DSM 62474 oder DSM 62475 oder deren Enzymen fermentiert.

## Claims

1. Process for the production of a mixture of (1S, 2S, 4R)-p-menth-8-en-1,2-diol and (1R, 2R, 4S)-p-menth-8-en-1,2-diol characterized in that racemic limonene is fermented with fungi of the genera Fusarium, Gibberella, Chaetomium, Diplodia, Corynespora, Glomerella, Aspergillus, Pestalotia, Streptomyces, Botryodiplodia or Botryosphaeria producing p-menth-8-en-1,2-diol or with their enzymes and that the said mixture is obtained.

2. Process for the production of (1S, 2S, 4R)-p-menth-8-en-1,2-diol, characterized in that (R)(+)-limonene is fermented with fungi of the genera Fusarium, Gibberella, Chaetomium, Diplodia, Corynespora, Glomerella, Aspergillus, Pestalotia, Streptomyces, Botryodiplodia or Botryosphaeria producing p-menth-8-en-1,2-diol or with their enzymes and that the said diol is obtained.

3. Process for the production of (1R, 2R, 4S)-p-menth-8-en-1,2-diol, characterized in that (S)(-)-limonene is fermented with fungi of the genera Fusarium, Gibberella, Chaetomium, Diplodia, Corynespora, Glomerella, Aspergillus, Pestalotia, Streptomyces, Botryodiplodia or Botryosphaeria producing p-menth-8-en-1,2-diol or with their enzymes and that the said diol is obtained.

4. Process according to claim 1, 2 or 3, characterized in that the fermentation is carried out with Diplodia gossypina ATCC-10936 or Corynespora cassiicola DSM 62474 or DSM 62475 or with their enzymes.

## Revendications

1. Procédé pour préparer un mélange de p-menthène-8 diol-1,2 (1S, 2S, 4R) et de p-menthène-8 diol-1,2 (1R 2R, 4S), procédé caractérisé en ce qu'on fait fermenter le limonène racémique avec des mycètes générateurs de p-menthène-8 diol-1,2 qui appartiennent aux genres Fusarium, Gibberella, Chaetomium, Diplodia, Corynespora, Glomerella, Aspergillus, Pestalotia, Streptomyces, Botryodiplodia et Botryosphaeria, ou avec leurs enzymes, et on isole le mélange cité.

2. Procédé de préparation du p-menthène-8 diol-1,2 (1S, 2S, 4R), caractérisé en ce qu'on fait fermenter le (+)-limonène-(R) avec des mycètes générateurs de p-menthène-8-diol-1,2 des genres Fusarium, Gibberella, Chaetomium, Diplodia, Corynespora, Glomerella, Aspergillus, Pestalotia, Streptomyces, Botryodiplodia et Botryosphaeria, ou avec leurs enzymes, et on isole le diol cité.

3. Procédé de préparation du p-menthène-8 diol-1,2 (1R, 2R, 4S), caractérisé en ce qu'on fait fermenter le (-)-limonène-(S) avec des mycètes générateurs de p-menthène-8 diol-1,2 des genres Fusarium, Gibberella, Chaetomium, Diplodia, Corynespora, Glomerella, Aspergillus, Pestalotia, Streptomyces, Botryodiplodia et Botryosphaeria, ou avec leurs enzymes, et on isole le diol cité.

4. Procédé selon l'une quelconque des revendications 1, 2 et 3, caractérisé en ce qu'on fait fermenter avec Diplodia gossypina ATCC 10936 ou Corynespora cassiicola DSM 62474 ou DSM 62475 ou avec leurs enzymes. 1